# EUROPEAN PATENT APPLICATION

(11) **EP 4 478 145 A1**
(43) Date of publication of application: **18.12.2024**
(21) Application number: 24182168.5
(22) Date of filing: 14.06.2024
(51) Int. Cl.: G05B 23/02, G06N 5/022, G06N 5/04, G06N 20/00

(54) **DIGITIZED REPORTS OF TECHNICAL SYSTEMS**

(30) Priority: 15.06.2023 US 202363508490 P
(71) Applicant: BeaconMedaes LLC, Rock Hill, South Carolina 29730 (US)
(72) Inventor: LEVINE, Benjamin, Indian Land, 29707 (US); MILLER, Melody, York, 29745 (US)
(74) Representative: Van Minnebruggen, Ewan Benito Agnes

(57) **Abstract**

A method, computer program product, and computer system for converting controller data uploaded to the cloud to digital report stored on the cloud and accessible via an online portal. One or more data packets from a controller of a compressor are received over a network, the one or more data packets including data associated with technical parameters of the compressor at a given state of the compressor. The data contained in the data packets are converted to a digital report of the given state of the compressor in a human readable format. The digital reports are stored in one or more databases accessible through an online portal associated with the computer system. One or more artificial intelligence models and machine learning techniques are leveraged to improve failure event predicting and corrective action response time for technical system, using the digital reports as input datasets.

## Description

### CROSS-REFERENCE TO RELATED APPLICATIONS

This application claims the benefit of and priority to U.S. Provisional Application No. 63/508,490, filed June 15, 2024, and entitled "Digitized Reports of Technical Systems."

### FIELD OF TECHNOLOGY

The following relates to digitized reports of technical systems, and more specifically, to converting controller data uploaded to the cloud to a digital report stored on the cloud and accessible via an online portal. Further, the digitized reports leverage artificial intelligence to improve technical systems.

### BACKGROUND

In places where industrial equipment or safety-critical equipment is used, such as a medical gas compressor used in a hospital, regular monitoring of the equipment is required in most jurisdictions, and at least preferred in all jurisdictions.

### SUMMARY

Embodiments of the present invention provide a method, a computer program product, and a computer system, for converting controller data uploaded to the cloud to digital report stored on the cloud and accessible via an online portal.

One or more data packets from a controller of a compressor are received over a network, the one or more data packets including data associated with technical parameters of the compressor at a given state of the compressor. The data contained in the data packets are converted to a digital report of the given state of the compressor in a human readable format. The digital reports are stored in one or more databases accessible through an online portal associated with the computer system. One or more artificial intelligence models and machine learning techniques can be leveraged to improve technical systems, such as failure event predicting and corrective action response time for technical system, using the digital reports as input datasets.

### BRIEF DESCRIPTION OF THE DRAWINGS

FIG. 1 depicts a computing environment which contains an example of an environment for the execution of at least some of the computer code involved in performing the inventive methods, in accordance with embodiments of the present invention.
FIG. 2 is a block diagram of modules included in code included in the system of FIG. 1, in accordance with embodiments of the present invention.
FIG. 3 is a flow chart of an embodiment of a method for converting controller data to a digital report, in accordance with embodiments of the present invention.
FIG. 4 is a flowchart of a process of predicting failure events and taking corrective action, where the operations of the flowchart are performed by one or more of the modules in FIG. 2, in accordance with embodiments of the present invention.
FIG. 5 is a flowchart of a process of simulating changes to a compressor system based on an output of a trained AI model, where the operations of the flowchart are performed by one or more of the modules in FIG. 2, in accordance with embodiments of the present invention.
FIG. 6 is a flowchart of a process of controlling a compressor system based on an output of a trained AI model, where the operations of the flowchart are performed by one or more of the modules in FIG. 2, in accordance with embodiments of the present invention.

### DETAILED DESCRIPTION

### OVERVIEW

In places where industrial equipment or safety-critical equipment is used, such as a medical gas compressor used in a hospital, regular monitoring of the equipment is required in most jurisdictions, and at least preferred in all jurisdictions. To comply with the monitoring requirements, organizations using the equipment employ trained technicians who visit the equipment on a daily basis, check for alarms, document sensor reading levels, and perform a general inspection based on an equipment-specific checklist. Currently, there is no standardized inspection and documentation method across, and organizations develop their own method to inspect the equipment and to document the inspection. For example, clipboards can be attached to each piece of equipment with places for technicians to sign off that the equipment was inspected on a given time/day. Another example is a single checklist that covers all pieces of equipment that is manually signed. Occasionally, organizations are audited to provide evidence of compliance with the monitoring requirements. Locating a complete record of inspection is often time consuming and sometimes not possible given the method of documenting inspections. Moreover, current inspection methods omit various technical parameters which are needed to accurately assess the operating status of the equipment at a current time or in the foreseeable future.

Embodiments of the present invention standardize and improve conventional inspection and documenting methods using an online portal accessible by members of the organization using industrial equipment or safety-critical equipment that requires regular monitoring. Data packets containing data associated with technical parameters are uploaded directly from a controller/computer system of the equipment over a network to one or more servers in the cloud over regular intervals, which convert the data in the data packets to a digital report of a state/status of the equipment. To trigger the upload of the data packets, a technician physically interacts with a graphical user interface (GUI) to log into an online portal associated with the one or more servers and navigates the GUI to upload the data packets automatically to the cloud. Requiring the physical interaction with the GUI ensures that a technician is present to initiate the upload. The data packets received by the one or more servers in the cloud are converted from key-value pairs into a digital report that is in a human readable form. The digital report includes a timestamp of the upload and various sensor readings, alarm statuses, unit running times, name of technician, location of the equipment, and the like. Members of the organization can access the digital reports stored on a cloud server from local machines via an online platform, such as MyMedGas^{®}, which operates as central digital location to manage medical assets, inventory, and teams.

Further, embodiments of the present invention improve machine learning algorithms used for predicting failure events of the equipment, simulating corrective actions, and determining corrective actions to implement on the equipment. A trained artificial intelligence (AI) model is used to output a predicted failure event of the equipment and a corrective action to take to avoid the failure event; the data from the data packets automatically uploaded from the equipment controller and/or the data from the digital report are used as inputs for the trained AI model. The trained AI model is trained by feeding datasets pertaining to actual failure events of the equipment and known solutions to correct or avoid the failure event. The output of the trained AI model is used to fix, modify, repair, change, etc. the equipment before the failure event occurs, or worsens.

Additionally, the output of the trained AI model is used to augment or otherwise change the GUI of the equipment controller to display a new icon indicative of a potential failure event. The change in the GUI alerts a technician to the potential problem, and physical interaction with the new icon launches a recommended solution to be problem which can be downloaded to the equipment controller or other computer from the one or more servers in the cloud that leverages the trained AI model for the solution. To ensure the detected potential failure event does not go unnoticed or ignored, a transmission of new data packets (e.g. at the next interval) from the equipment controller can be blocked unless a confirmation is received from the equipment controller that a physical input associated with the new icon of the graphical user interface was received; a notification to the online portal that the transmission of new data packets was blocked is immediately sent. Alternatively, the transmission of new data packets for a new state of the compressor can be accepted and a digital report created but the equipment controller may display on the graphical user interface that the transmission was blocked.

Further, an output of the trained AI model is fed to a digital computer model of the compressor for simulating adjustments of parameters of the compressor/medical air system before implementing the adjustments to an active compressor located at the facility.

In further embodiments, an output of the trained AI model is used to automatically control an active compressor located at the facility by implanting a suggested change.

### COMPUTING ENVIRONMENT

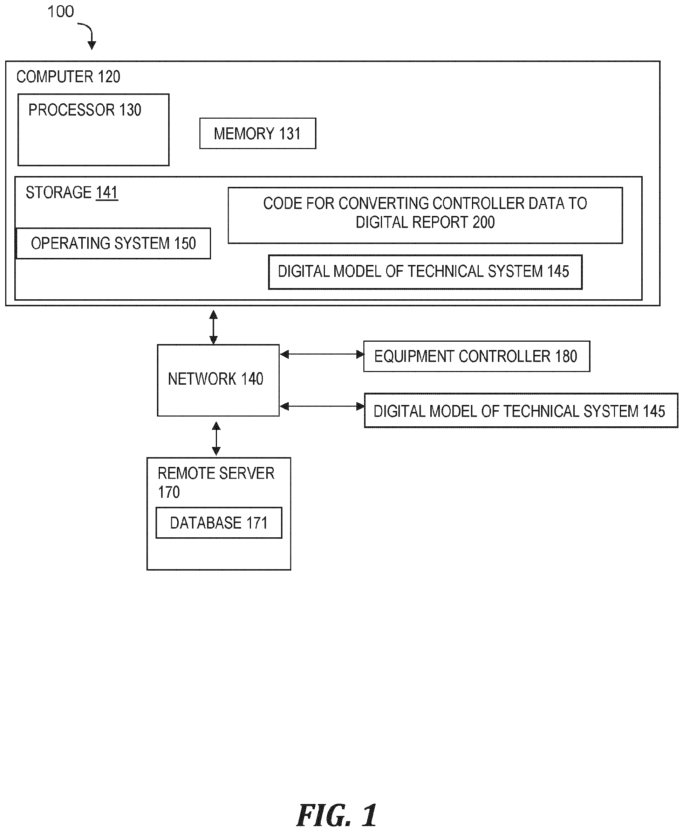

FIG. 1 depicts a computing environment which contains an example of an environment for the execution of at least some of the computer code involved in performing the inventive methods, in accordance with embodiments of the present invention. Computing environment 100 contains an example of an environment for the execution of at least some of the computer code involved in performing the inventive methods, such as code 200 for converting controller data to digital report. Computing environment 100 includes, for example, computer 120, network 140, such as a wide area network (WAN), equipment controller 180, and remote server 170 with database 171. Computer 120 includes processor 130, memory 131, storage 141 including operating system 150 and code 200. The computer 120 is connected to the remote server 170, the equipment controller 180, and a digital model of a technical system 145 over the network 140. The digital model of a technical system 145 may alternatively or additionally be located in storage 141.

Cloud computing is a model of service delivery for enabling convenient, on-demand network access to a shared pool of configurable computing resources (e.g., networks, network bandwidth, servers, processing, memory, storage, applications, virtual machines, and services) that can be rapidly provisioned and released with minimal management effort or interaction with a provider of the service. This cloud model may include at least five characteristics, at least three service models, and at least four deployment models.

Characteristics are as follows:
*On-demand self-service*: a cloud consumer can unilaterally provision computing capabilities, such as server time and network storage, as needed automatically without requiring human interaction with the service's provider.
*Broad network access*: capabilities are available over a network and accessed through standard mechanisms that promote use by heterogeneous thin or thick client platforms (e.g., mobile phones, laptops, and PDAs).
*Resource pooling*: the provider's computing resources are pooled to serve multiple consumers using a multi-tenant model, with different physical and virtual resources dynamically assigned and reassigned according to demand. There is a sense of location independence in that the consumer generally has no control or knowledge over the exact location of the provided resources but may be able to specify location at a higher level of abstraction (e.g., country, state, or datacenter).
*Rapid elasticity*: capabilities can be rapidly and elastically provisioned, in some cases automatically, to quickly scale out and rapidly released to quickly scale in. To the consumer, the capabilities available for provisioning often appear to be unlimited and can be purchased in any quantity at any time.
*Measured service*: cloud systems automatically control and optimize resource use by leveraging a metering capability at some level of abstraction appropriate to the type of service (e.g., storage, processing, bandwidth, and active user accounts). Resource usage can be monitored, controlled, and reported, providing transparency for both the provider and consumer of the utilized service.

Service Models are as follows:
*Software as a Service (SaaS)*: the capability provided to the consumer is to use the provider's applications running on a cloud infrastructure. The applications are accessible from various client devices through a thin client interface such as a web browser (e.g., web-based e-mail). The consumer does not manage or control the underlying cloud infrastructure including network, servers, operating systems, storage, or even individual application capabilities, with the possible exception of limited user-specific application configuration settings.
*Platform as a Service (PaaS)*: the capability provided to the consumer is to deploy onto the cloud infrastructure consumer-created or acquired applications created using programming languages and tools supported by the provider. The consumer does not manage or control the underlying cloud infrastructure including networks, servers, operating systems, or storage, but has control over the deployed applications and possibly application hosting environment configurations.
*Infrastructure as a Service (IaaS)*: the capability provided to the consumer is to provision processing, storage, networks, and other fundamental computing resources where the consumer is able to deploy and run arbitrary software, which can include operating systems and applications. The consumer does not manage or control the underlying cloud infrastructure but has control over operating systems, storage, deployed applications, and possibly limited control of select networking components (e.g., host firewalls).

Deployment Models are as follows:
*Private cloud*: the cloud infrastructure is operated solely for an organization. It may be managed by the organization or a third party and may exist on-premises or off-premises.
*Community cloud*: the cloud infrastructure is shared by several organizations and supports a specific community that has shared concerns (e.g., mission, security requirements, policy, and compliance considerations). It may be managed by the organizations or a third party and may exist on-premises or off-premises.
*Public cloud*: the cloud infrastructure is made available to the general public or a large industry group and is owned by an organization selling cloud services.
*Hybrid cloud*: the cloud infrastructure is a composition of two or more clouds (private, community, or public) that remain unique entities but are bound together by standardized or proprietary technology that enables data and application portability (e.g., cloud bursting for load-balancing between clouds).

A cloud computing environment is service oriented with a focus on statelessness, low coupling, modularity, and semantic interoperability. At the heart of cloud computing is an infrastructure that includes a network of interconnected nodes.

Cloud computing environment includes one or more cloud computing nodes with which local computing devices used by cloud consumers, such as, for example, personal digital assistant (PDA) or cellular telephone, desktop computer, laptop computer, etc. Nodes may communicate with one another. They may be grouped (not shown) physically or virtually, in one or more networks, such as Private, Community, Public, or Hybrid clouds as described hereinabove, or a combination thereof. This allows cloud computing environment to offer infrastructure, platforms and/or software as services for which a cloud consumer does not need to maintain resources on a local computing device. It is understood that the types of computing devices are intended to be illustrative only and that computing nodes and cloud computing environment can communicate with any type of computerized device over any type of network and/or network addressable connection (e.g., using a web browser).

A cloud computing environment includes a set of functional abstraction layers. The following layers and corresponding functions are provided:

Hardware and software layer includes hardware and software components. Examples of hardware components include: mainframes; RISC (Reduced Instruction Set Computer) architecture-based servers; servers; blade servers; storage devices; and networks and networking components. In some embodiments, software components include network application server software and database software.

Virtualization layer provides an abstraction layer from which the following examples of virtual entities may be provided: virtual servers; virtual storage; virtual networks, including virtual private networks; virtual applications and operating systems; and virtual clients.

In one example, management layer may provide the functions described below. Resource provisioning provides dynamic procurement of computing resources and other resources that are utilized to perform tasks within the cloud computing environment. Metering and Pricing provide cost tracking as resources are utilized within the cloud computing environment, and billing or invoicing for consumption of these resources. In one example, these resources may include application software licenses. Security provides identity verification for cloud consumers and tasks, as well as protection for data and other resources. User portal provides access to the cloud computing environment for consumers and system administrators. Service level management provides cloud computing resource allocation and management such that required service levels are met. Service Level Agreement (SLA) planning and fulfillment provides pre-arrangement for, and procurement of, cloud computing resources for which a future requirement is anticipated in accordance with an SLA.

Workloads layer provides examples of functionality for which the cloud computing environment may be utilized. Examples of workloads and functions which may be provided from this layer include: mapping and navigation; software development and lifecycle management; virtual classroom education delivery; data analytics processing; transaction processing; and digital report creation and failure event prediction and corrective action.

It is to be understood that although this disclosure includes a detailed description on cloud computing, implementation of the teachings recited herein are not limited to a cloud computing environment. Rather, embodiments of the present invention are capable of being implemented in conjunction with any other type of computing environment now known or later developed.

### SYSTEM AND PROCESS FOR CONVERTING CONTROLLER DATA TO DIGITAL REPORT

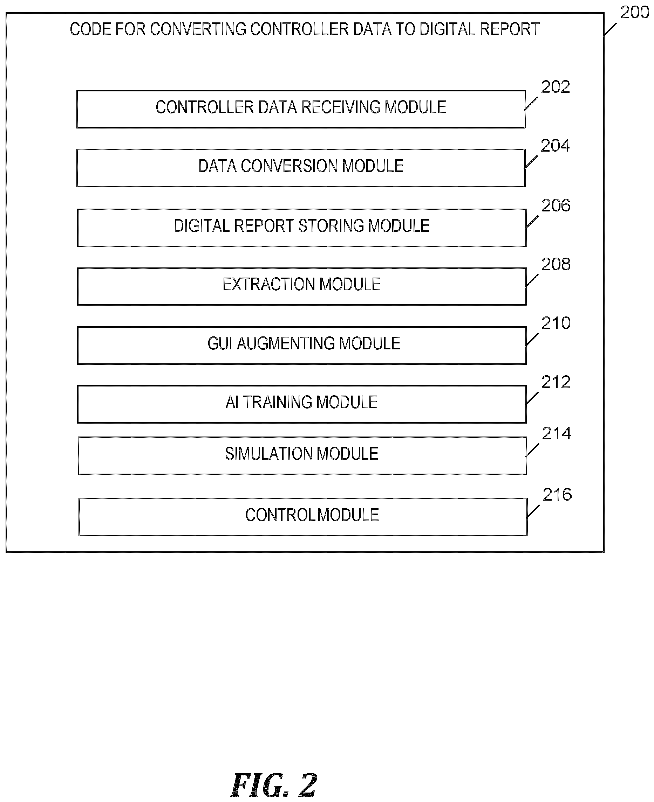

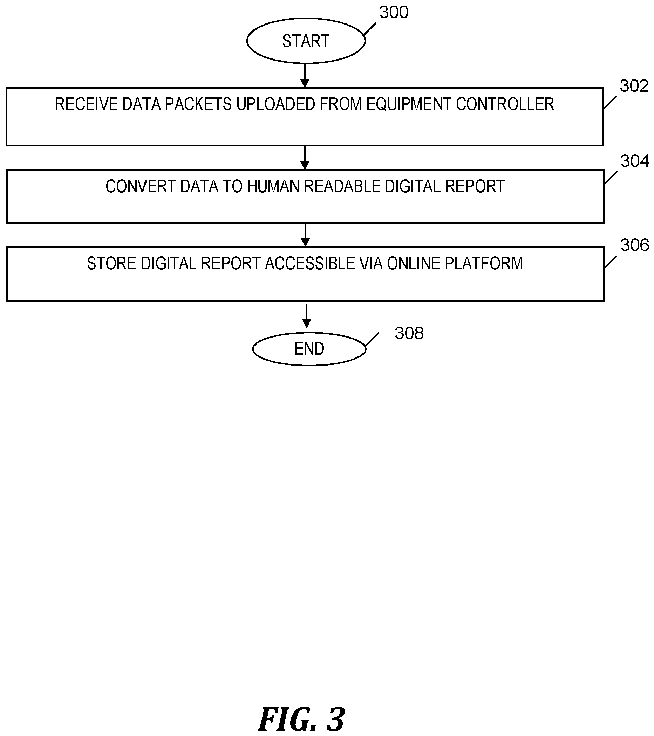

FIG. 2 is a block diagram of modules included in code included in the system of FIG. 1, in accordance with embodiments of the present invention. Code 200 includes a controller data receiving module 202, a data conversion module 204, a digital report storing module 206, an extraction module 208, a GUI augmenting module 210, an AI training module 212, a simulation module 214, and a control module 216. The number of modules can vary, and some modules may be combined with other modules or separated into two or more modules. The functionality of the modules included in code 200 is discussed in detail in the discussion of FIG. 3, which is presented below.

FIG. 3 is a flowchart of a process of converting controller data to a digital report, where the operations of the flowchart are performed by one or more of the modules in FIG. 2, in accordance with embodiments of the present invention. The process of FIG. 3 begins at a start node 300.

In step 302, controller data receiving module 202 (see FIG. 2) receives one or more data packets from an equipment controller over a network. In an embodiment, the one or more data packets are received at regular intervals (e.g. the equipment controller uploads new data packets every hour). An equipment controller can be a controller or on-board computer of the equipment, or one or more processors of a computing system associated with the equipment. The equipment can be industrial or safety-critical equipment, such as a medical gas compressor system used in hospitals. The equipment can be a technical system which is comprised of one or more technical components, parts, systems, subsystems, and the like. Examples of a technical system are a compressor system, a multistage compressor, a vacuum pump, medical gas delivery system, or any mechanical-based system that requires regular monitoring and/or maintenance. The one or more data packets include data associated with technical parameters of the equipment at a given state of the equipment, derived from one or more sensors, alarm circuity, and the like, of the equipment. The technical parameters include, but are not limited to a system pressure, an ambient temperature, a compressor discharge temperature, a line pressure, a speed of a variable speed drive, a dew point, a carbon monoxide level, a carbon dioxide level, an operating mode of a component of the compressor, a running time of the component, average daily runtime of the compressor, unit status, an alarm status, voltage output, and voltage input. The technical parameters included in the digital report can vary by equipment, manufacturer, state and local laws, and hospital.

In step 304, the data conversion module 204 converts the data contained in the data packets to a digital report of the given state of the compressor in a human readable format. The data contained in the one or more data packets are key-value pairs, indicating a key associated with a technical parameter and a value associated with the technical parameter. The data can be converted in a way that semantically matches a custom template of an organization using natural language processing tools and key-value matching. A text of the digital report so that the information is presented as continuous natural language that can be parsed by conventional natural language processing algorithms and machine reading and comprehension engines.

In step 306, the digital report storing module 206 stored the digital report in one or more databases accessible through an online portal associated with the computer system. Members of an organization can log in to view, retrieve, or otherwise access the digital reports at any time from local machines over a network. Furthers, members logged into the online platform can query the database(s) in natural language and the report storing module 206 can retrieve relevant digital reports.

### SYSTEM AND PROCESS FOR PREDICTING FAILURE EVENTS USING TRAINED AI MODEL

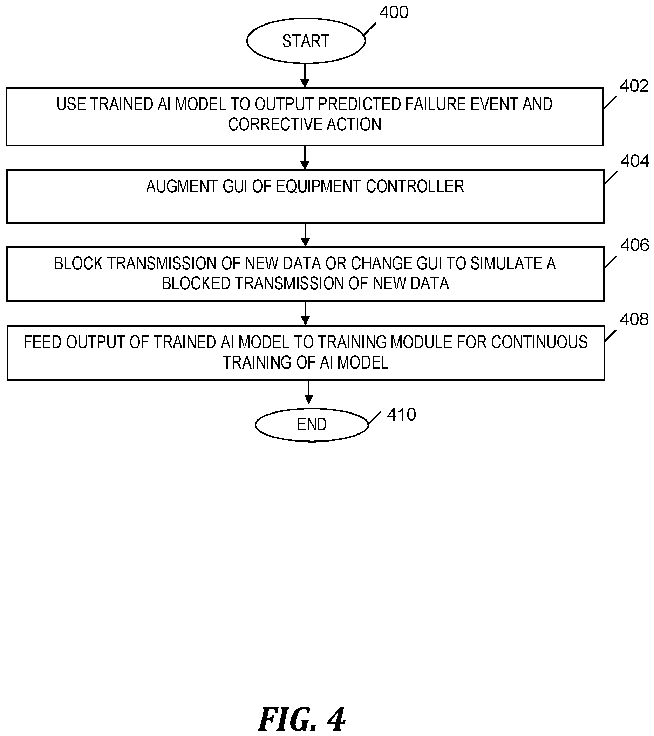

FIG. 4 is a flowchart of a process of predicting failure events and taking corrective action, where the operations of the flowchart are performed by one or more of the modules in FIG. 2, in accordance with embodiments of the present invention. The process of FIG. 4 begins at a start node 400 and ends at end node 410.

In step 402, the extraction module 208 uses a trained artificial intelligence model to extract, from the one or more data packets, a predicted failure event of the compressor and a corrective action, by inputting the data from the one or more data packets transmitted by the controller of the compressor.

In step 404, the GUI augmenting module 210, augments the graphical user interface of the controller to display a new icon indicative of a potential failure event, in response to a trained artificial intelligence model outputting a predicted failure event based on the one or more datasets being fed into the trained artificial intelligence model by the one or more processors.

In step 406, the GUI augmenting module 210 blocks a transmission of new data packets for a new state of the compressor from the controller of the compressor unless a confirmation is received from the controller of the compressor that a physical input associated with the new icon of the graphical user interface was received, and sends a notification to the online portal that the transmission of new data packets was blocked.

Alternatively, the GUI augmenting module 210 accepts a transmission of new data packets for a new state of the compressor from the controller of the compressor for preparing a new digital report, wherein, as a function of the accepting, the one or more processors communicate to the controller to display on the graphical user interface that the transmission was blocked, unless a confirmation is received from the controller of the compressor that a physical input associated with the new icon of the graphical user interface was received by the controller

In step 408, the AI training module 214 feeds the output of the trained AI model into the training of the AI model for continuous training of the AI model.

### SYSTEM AND PROCESS FOR SIMULATING CHANGES TO TECHNICAL SYSTEM USING OUTPUT OF TRAINED AI MODEL

Output of a trained AI model can be fed to a digital computer model of the compressor for simulating adjustments of parameters of the compressor/medical air system before implementing the adjustments to an active compressor located at the facility. In most cases, remotely implementing changes to an active compressor system is not ideal due to the active compressor system being used by patients. Instead, a digital computer model of the compressor system is created to simulate changes and/or adjustments to a compressor system provided by the trained AI model. For example, the trained AI model can suggest changes to the active compressor system based on the trained AI model's analysis of the digital reports to improve the operation/functioning of the active compressor system. Prior to implementing a change to the active compressor system, the suggested changes to one or more operational parameters are simulated in the digital model of the compressor system. The simulation determines the impact of the suggested changes and can also determine the optimal parameters for the active compressor system. In an exemplary embodiment, the simulation can determine the most efficient method of operating the compressor system's compressors/vacuum pumps in order to minimize run time therefore saving energy and wear on the compressors/pumps. The results of the simulation can be made accessible via the online portal for assessment and implementation to the active compressor system.

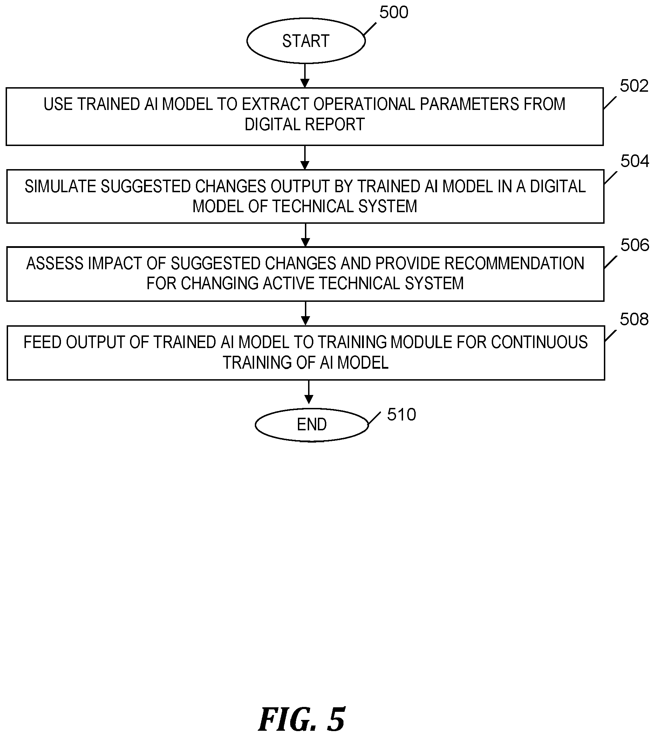

FIG. 5 is a flowchart of a process of simulating changes to a compressor system based on an output of a trained AI model, where the operations of the flowchart are performed by one or more of the modules in FIG. 2, in accordance with embodiments of the present invention. The process of FIG. 5 begins at a start node 500 and ends at end node 510.

In step 502, the extraction module 208 uses a trained artificial intelligence model to extract, from the one or more data packets, operational parameters of an active compressor system, by inputting the data from the one or more data packets transmitted by the controller of the compressor. The extraction module 208 feeds the digital report as input into the trained AI model, which extracts the operational/technical parameters of the active compressor system and assesses an efficiency of the active compressor by comparing one or more operational/technical parameters against one or more other operational/technical parameters of the active compressors. The output of the trained AI model is one or more suggested changes to one or more operational/technical parameters of the active compressor system.

As an example, the trained AI model compares an amount of running time for individual compressors based on an operating pressure of the system to determine a flow demand, and determines that waiting to turn on additional compressors until a lower operating pressure would result in sustaining a desired operating pressure while running fewer units. The trained AI model outputs a suggestion to lower pressure band settings while maintaining the same system output pressure to reduce power consumption and excess wear on the compressors. Many different scenarios can be analyzed by the trained AI model.

In step 504, the simulation module 214 simulates the suggested change(s) in the digital model of the technical system 145 that were suggested by the trained AI model in step 502.

In step 506, the simulation module 214 assesses the impact of the changes in the digital model 145. Continuing the example above, the simulation module 214 lowers pressure band settings while maintaining the same system output pressure and measures the impact to other operational/technical parameters of the digital model of the active compressor system. If the values of the other operational/technical parameters are unaffected by the change, the simulation module 214 recommends the suggested change via the online portal. Conversely, if the values of the other operational/technical parameters are adversely affected by the change, the simulation module 214 does not recommend the suggested change, and may provide a record in the online portal.

In step 508, the AI training module 214 feeds the recommendation and/or results of the simulation back into the trained AI model for continuous training of the AI model.

### SYSTEM AND PROCESS FOR CONTROLLING A TECHNICAL SYSTEM USING OUTPUT OF TRAINED AI MODEL

Output of the trained AI model is used to control an active compressor located at the facility by implanting a suggested change. A trained AI model can suggest changes to the active compressor system based on the trained AI model's analysis of the digital reports to improve the operation/functioning of the active compressor system. One or more suggested changes to one or more operational parameters are implemented by sending a control signal to an equipment controller, which controls one or more physical components of the compressor system. simulated in the digital model of the compressor system. A record of the change can be made accessible via the online portal.

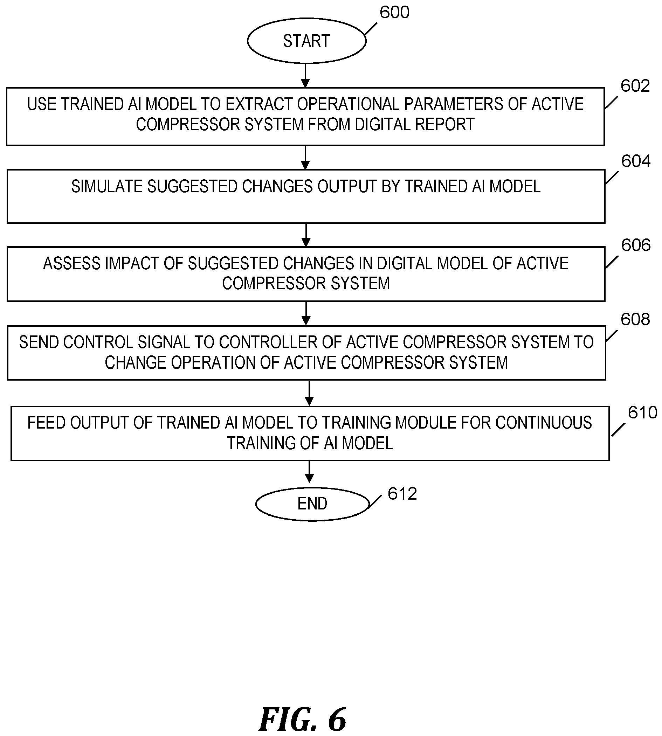

FIG. 6 is a flowchart of a process of controlling a compressor system based on an output of a trained AI model, where the operations of the flowchart are performed by one or more of the modules in FIG. 2, in accordance with embodiments of the present invention. The process of FIG. 6 begins at a start node 600 and ends at end node 612.

In step 602, the extraction module 208 uses a trained artificial intelligence model to extract, from the one or more data packets, operational parameters of an active compressor system, by inputting the data from the one or more data packets transmitted by the controller of the compressor. The extraction module 208 feeds the digital report as input into the trained AI model, which extracts the operational/technical parameters of the active compressor system and assesses an efficiency of the active compressor by comparing one or more operational/technical parameters against one or more other operational/technical parameters of the active compressors. The output of the trained AI model is one or more suggested changes to one or more operational/technical parameters of the active compressor system. As another example, the trained AI Model compares a time between switching dryer towers and determines that the system does not need to purge as much air in order to still maintain the ability for the desiccant to absorb the required moisture. The trained AI model output suggests reducing the purge time for the dryer towers. This allows the system to lose less compressed air meaning that the compressors do not have to run as much as they are currently running, saving energy consumed by the system and excess wear on the compressors. Many different scenarios can be analyzed by the trained AI model.

In step 604, the simulation module 214 simulates the suggested change(s) in the digital model of the technical system 145 that were suggested by the trained AI model in step 502.

In step 606, the simulation module 214 assesses the impact of the changes in the digital model 145. Continuing the example above, the simulation module 214 reduces the purge time for the dryer towers and measures the impact to other operational/technical parameters of the digital model of the active compressor system. If the values of the other operational/technical parameters are unaffected by the change, the simulation module 214 recommends the suggested change. Conversely, if the values of the other operational/technical parameters are adversely affected by the change, the simulation module 214 does not recommend the suggested change, and may provide a record in the online portal.

In step 608, the control module 216 receives the recommended change from the simulation module 214 and generates a control signal and sends the control signal to the equipment controller 180 over network 140. The control signal is one or more electronic signals that instruct the equipment controller to effectuate a change to the active compressor system. The equipment controller 180 sends a control/command signal to one or more physical components (e.g., valves, actuators, drive units, onboard computers, etc.) of the active compressor system which changes a technical parameter of the active component system.

In one example, in order for the active compressor system to dryer the air, it passes air through alternating chambers of desiccant. To dryer the inactive chamber, some of the dried air is passed through the desiccant and purged into the equipment room to regenerate the desiccant. The trained AI model detects, from the digital report, that a system appeared to have very little dryer activity, and determines that an extra actuator could be activated to prevent purging as much air as normal. Saving some of this air means the compressors have to run less frequently because they are not wasting some of the compressed air into the equipment room. Thus, the control module 216, in response to the output of the trained AI model, sends a control signal to the equipment controller 180 to actuate an actuator responsible for purging air. Further, the trained AI model can learn that the timing of a purge cycle on the dryer could also be reduced so it spends less time purging. The control module 216 can prepare a software update to push to the equipment controller 180 of the active compressor system that would affect the timing variables for the dryer.

In another example, while the compressor is running, there is a valve actuated on an output of a discharge radiator to make sure that some of the moisture that is generated by the cooling of the air is periodically blown to a drain. This prevents excess moisture from entering the pressure vessel and possibly turning into humidity that needs to be removed by the dryers. The trained AI model determines from the digital report that a system is not generating nearly as much heat because it is not running as frequently. Thus, the trained AI model suggests that the timing of an actuator valve could be reduced in time and frequency so that less pressurized air is released from the equipment meaning that the running time of the compressors will be reduced. The control module 216 sends a control signal to the equipment controller 180 to actuate the valve, thereby controlling the compressor system.

In another example, the starting and stopping of compressors and vacuum pumps (units) is controlled by pressure bands where units cut on additionally as the pressure drops and cut off as the pressure rises. The trained AI model, in response to being fed the digital report, learns the regulator flow of the customer will cause the system to turn units on and off more than they would have to if the pressure band were adjusted slightly to optimize how many units need to actually start if the regular load can be handled by one less unit. The trained AI model can model different pressure bands against the regular flow of the system to determine an optimal pressure band setting for the system. The optimal pressure band setting for the system could be implemented by the control module 216 over network 140 to automatically control variables in the system. The new pressure bands applied to the system could be analyzed to see if the change improved the system because the digital report would indicate that the average daily run time of the compressors would decrease.

In some embodiments, steps 604 and 606 (i.e. simulation) are optional such that the suggested changes output by the trained AI model are not first simulated before being implemented into an active compressor system. The suggested changes could be implemented if a confidence that the change will pose no threat to the operation of the active compressor is above a predetermined threshold.

In step 610, the AI training module 214 feeds the applied changes back into the trained AI model for continuous training of the AI model.

### TRAINING THE AI MODEL FOR PREDICTING COMPONENT FAILURE

A training module 212 of code 200 trains the first AI model using supervised machine learning or unsupervised machine learning. In an exemplary embodiment, the model's algorithm is trained to predict failure events and taking corrective action.

Input samples (e.g. digital report(s)) are fed into one or more neural networks to obtain a first vector and a second vector. An example of a first input sample is a volume air purged from the system, and the second input sample is an average system pressure. Another example of a first input sample is an alarm status, and the second input sample is a temperature of the system. Another example of a first input sample is a moisture content associated with a discharge radiator and the second input sample is a pressure associated with the discharge radiator. A number between 0 and 1 is calculated using a cosine similarity function of the first vector and the second vector. The number closer to 1 indicates a match between the sample types, and the number closer to 0 indicates that the sample types do not match. A knowledge graph using the relative relationship of the at least two inputs is built and translated into readable text to understand impact the life of a component of the compressor system. The inputs can be represented on the knowledge graph, along with the relative relationship to each other. The knowledge graph is translated into machine readable natural language using a natural language generation method based on data of the knowledge graph in the graph form to organize a semantic information of each node of the knowledge graph into a continuous natural language temporary text, which is then translated into the machine-readable natural language using a natural language style transfer method. In this way, the second AI model is trained to learn what, if any, components may fail within a predefined window of time.

### TRAINING THE AI MODEL FOR SIMULATING AND/OR CONTROLLING COMPRESSOR SYSTEM

A training module 212 of code 200 trains the first AI model using supervised machine learning or unsupervised machine learning. In an exemplary embodiment, the model's algorithm is trained to determine how the compressor system can be controlled to improve efficiency and/or avoid failure.

Input samples (e.g. digital report(s)) are fed into one or more neural networks to obtain a first vector and a second vector. An example of a first input sample is an average daily runtime of the vacuum pump, and the second input sample is an average system pressure. Another example of a first input sample is a speed of a variable speed drive, and the second input sample is an average daily runtime of the variable speed drive. Another example of a first input sample is a moisture content associated with a discharge radiator and the second input sample is a temperature associated with the discharge radiator. A number between 0 and 1 is calculated using a cosine similarity function of the first vector and the second vector. The number closer to 1 indicates a match between the sample types, and the number closer to 0 indicates that the sample types do not match. A knowledge graph using the relative relationship of the at least two inputs is built and translated into readable text to understand impact the life of a component of the compressor system. The inputs can be represented on the knowledge graph, along with the relative relationship to each other. The knowledge graph is translated into machine readable natural language using a natural language generation method based on data of the knowledge graph in the graph form to organize a semantic information of each node of the knowledge graph into a continuous natural language temporary text, which is then translated into the machine-readable natural language using a natural language style transfer method. In this way, the second AI model is trained to learn what, if any, corrective actions can be taken to improve the efficiency of the compressor system.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A computer-implemented method comprising:
receiving, by one or more processors of a computer system, one or more data packets from a controller of a compressor over a network, the one or more data packets including data associated with technical parameters of the compressor at a given state of the compressor;
converting, by the one or more processors, the data contained in the data packets to a digital report of the given state of the compressor in a human readable format; and
storing, by the one or more processors, the digital report in one or more databases accessible through an online portal associated with the computer system.

2. The computer-implemented method of claim 1, further comprising:
using, by the one or more processors, a trained artificial intelligence model to extract, from the one or more data packets, a predicted failure event of the compressor and a corrective action, by inputting the data from the one or more data packets transmitted by the controller of the compressor.

3. The computer-implemented method of claim 1, further comprising:
training, by the one or more processors, the trained artificial intelligence model by feeding input datasets of actual failure events of the compressor and known solutions to avoid or correct the actual failure event into an artificial intelligence model to output a predicted failure event of the compressor and a corrective action to avoid the predicted failure event;
building, by the one or more processors, a knowledge graph using a relative relationship of at least two nodes of the input datasets of actual failure events; and
translating, by the one or more processors, the knowledge graph into machine readable natural language.

4. The computer-implemented method of claim 1, wherein the controller includes a graphical user interface that, only in response to a physical input, allows a transmission of the one or more data packets over the network to be received by the computer system.

5. The computer-implemented method of claim 4, further comprising:
augmenting, by the one or more processors, the graphical user interface of the controller to display a new icon indicative of a potential failure event, in response to a trained artificial intelligence model outputting a predicted failure event based on the one or more datasets being fed into the trained artificial intelligence model by the one or more processors.

6. The computer-implemented method of claim 5, further comprising:
blocking, by the one or more processors, a transmission of new data packets for a new state of the compressor from the controller of the compressor unless a confirmation is received from the controller of the compressor that a physical input associated with the new icon of the graphical user interface was received; and
sending, by the one or more processors, a notification to the online portal that the transmission of new data packets was blocked.

7. The computer-implemented method of claim 5, further comprising:
accepting, by the one or more processors, a transmission of new data packets for a new state of the compressor from the controller of the compressor for preparing a new digital report, wherein, as a function of the accepting, the one or more processors communicate to the controller to display on the graphical user interface that the transmission was blocked, unless a confirmation is received from the controller of the compressor that a physical input associated with the new icon of the graphical user interface was received by the controller.

8. The computer-implemented method of claim 1, wherein the converting the data from the one or more data packets to the digital report comprises formatting from one data file type to another data file type.

9. The computer-implemented method of claim 1, wherein the technical parameters of the compressor include a system pressure, an ambient temperature, a compressor discharge temperature a line pressure, a speed of a variable speed drive, a dew point, a carbon monoxide level, a carbon dioxide level, an operating mode of a component of the compressor, a running time of the component, average daily runtime of the compressor, unit status, an alarm status, voltage output, and voltage input.

10. The computer-implemented method of claim 1, wherein the data contained in the one or more data packets are key-value pairs, indicating a key associated with a technical parameter and a value associated with the technical parameter.

11. The computer-implemented method of claim 1, wherein the one or more data packets are received at predetermined intervals, and represent a new state of the compressor.

12. The computer-implemented method of claim 1, further comprising:
reconstructing, by the one or more processors, a text of the digital report so that the information is presented as continuous natural language that can be parsed by conventional natural language processing algorithms and machine reading and comprehension engines.

13. A computer program product comprising a computer readable hardware storage medium having program instructions embodied therewith, the program instructions readable by one or more processors of a computer system to cause the one or more processors to:
receive one or more data packets from a controller of a compressor over a network, the one or more data packets including data associated with technical parameters of the compressor at a given state of the compressor;
convert the data contained in the data packets to a digital report of the given state of the compressor in a human readable format; and
store the digital report in one or more databases accessible through an online portal associated with the computer system.

14. A computer system comprising:
one or more computer processors;
one or more computer readable storage media; and
computer readable code stored collectively in the one or more computer readable storage media, with the computer readable code including data and instructions to cause the one or more computer processors to perform at least the following operations:
receiving, by one or more processors of a computer system, one or more data packets from a controller of a compressor over a network, the one or more data packets including data associated with technical parameters of the compressor at a given state of the compressor;
converting, by the one or more processors, the data contained in the data packets to a digital report of the given state of the compressor in a human readable format; and
storing, by the one or more processors, the digital report in one or more databases accessible through an online portal associated with the computer system.

15. The computer system of claim 14, further comprising:
using, by the one or more processors, a trained artificial intelligence model to extract, from the one or more data packets, the predicted failure event of the compressor, by inputting the data from the one or more data packets transmitted by the controller of the compressor.

16. The computer system of claim 14, further comprising:
training, by the one or more processors, the trained artificial intelligence model by feeding input datasets of actual failure events of the compressor and known solutions to avoid or correct the actual failure event into an artificial intelligence model to output a predicted failure event of the compressor and a corrective action to avoid the predicted failure event;
building, by the one or more processors, a knowledge graph using a relative relationship of at least two nodes of the input datasets of actual failure events; and
translating, by the one or more processors, the knowledge graph into machine readable natural language.

17. The computer system of claim 14, wherein the controller includes a graphical user interface that, only in response to a physical input, allows a transmission of the one or more data packets over the network to be received by the computer system.

18. The computer system of claim 17, further comprising:
augmenting, by the one or more processors, the graphical user interface of the controller to display a new icon indicative of a potential failure event, in response to a trained artificial intelligence model outputting a predicted failure event based on the one or more datasets being fed into the trained artificial intelligence model by the one or more processors.

19. The computer system of claim 18, further comprising:
blocking, by the one or more processors, a transmission of new data packets for a new state of the compressor from the controller of the compressor unless a confirmation is received from the controller of the compressor that a physical input associated with the new icon of the graphical user interface was received; and
sending, by the one or more processors, a notification to the online portal that the transmission of new data packets was blocked.

20. The computer system of claim 19, further comprising:
accepting, by the one or more processors, a transmission of new data packets for a new state of the compressor from the controller of the compressor for preparing a new digital report, wherein, as a function of the accepting, the one or more processors communicate to the controller to display on the graphical user interface that the transmission was blocked, unless a confirmation is received from the controller of the compressor that a physical input associated with the new icon of the graphical user interface was received by the controller.
